(19) Europäisches Patentamt
European Patent Office
Office européen des brevets

(11) Publication number: **0 035 454 A2**

# (12) EUROPEAN PATENT APPLICATION

(21) Application number: 81400317.4

(22) Date of filing: 27.02.81

(51) Int. Cl.³: C 07 C 103/52, C 12 N 15/00, A 61 K 37/24, C 12 N 1/00 // C12R1/19

(30) Priority: 28.02.80 US 125685

(43) Date of publication of application: 09.09.81 Bulletin 81/36

(84) Designated Contracting States: AT BE CH DE FR GB IT LI LU NL SE

(71) Applicant: GENENTECH, INC., 460 Point San Bruno Boulevard, So. San Francisco California 94080 (US)

(72) Inventor: Crea, Roberto, 1520 Howard Avenue, Burlingame California 94010 (US)
Inventor: Wetzel, Ronald B., 76 Melrose, San Francisco California 94131 (US)

(74) Representative: Rinuy, Guy et al, 14, Avenue de la Grande Armée, F-75017 Paris (FR)

(54) **Desacetylthymosin alpha 1, its process of preparation and its use.**

(57) The present invention concerns desacetylthymosin alpha 1, an immune restorative polypeptide hormone. This polypeptide is synthesized in E. coli using recombinant DNA cloning techniques. Based on the known amino acid sequence, a gene coding for thymosin alpha 1 was designed and enzymatically assembled from chemically synthesized oligodeoxyribonucleotide fragments. The gene was ligated into plasmid pBR322, placed under lac operon control, and thymosin alpha 1 expressed as part of a $\beta$-galactosidase chimeric protein. Cyanogen bromide cleavage of this protein gave a mixture of polypeptides among which thymosin alpha 1 was deteoted by radioimmunoassay. The E. coli product is identical to thymosin alpha 1 isolated from calf thymus in amino acid sequence and exhibits similar activity in a radioimmunoassay.

The compound is hence a candidate for administration in effective immunopotentiating dosage in the treatment of immuno-depressed and immuno-suppressed conditions.

EP 0 035 454 A2

DESACETYLTHYMOSIN ALPHA 1, ITS PROCESS OF PREPARATION AND ITS USE.

This invention relates to the microbial expression of the polypeptide thymosin alpha 1 in non-acetylated form and to the polypeptide so produced.

The thymus gland produces a family of polypeptides termed thymosin. Publications referred to herein are incorporated by reference to illuminate the background of the invention and, to avoid repetitive citation, are grouped in the following section.

BIBLIOGRAPHIC SECTION

1. Hooper, J.A., McDaniel, M.C., Thurman, G.B., Cohen, G.H., Schulof, R.S. and Goldstein, A.L. 1975. Annals N.Y. Acad. Sci. 249, 125-144

2. Goldstein, A.L., Guha, A., Hardy, M.A. and White, A. (1972) Proc. Natl. Acad. Sci. USA 69, 1800-1803

3. Low, T.L.K. and Goldstein, A.L. in The Year in Hematology, 1978 (Silber, R.D., Gordon, A.S. and LoBue, J., eds) pp. 281-319, Plenum Press, New York

4. Goldstein, A.L., Low, T.L.K., McAdoo, M., McClure, J. Thurman, G.B., Rossio, J.L., Lai, C.-Y., Chang, D., Wang, S.-S., Harvey, C., Ramel, A.H., and Meienhofer, J. (1977) Proc. Natl. Acad. Sci. USA 24, 725-729

5. Low, T.L.K., Thurman, G.B., McAdoo, M., McClure, J., Rossio, J.L., Naylor, P.H. and Goldstein, A.L. (1979) J. Biol. Chem. 254, 981-986

6. Wara, D. and Ammann, A.J. (1978) Transplant. Proc. 10, 203-209

7. Goldstein, A.L., Thurman, G.B., Rossio, J.L., and Costanzi, J.J. (1977) Transplant Proc. 9, 1141-1144

8. Schafer, L.A., Gutterman, J.U., Hersh, E.M., Mavligit, G.M., and Goldstein, A.L. (1977) in Progress in Cancer Research and Therapy, Vol. 2(M.A. Chirigos, ed.) pp. 329-346, Raven Press, New York

9. Low, T.L.K. and Goldstein, A.L. (1979) J. Biol. Chem. 254, 987-995

10. Wang, S.-S., Kulesha, I.D., Winter, D.P. (1978) J. Amer. Chem. Soc. 101, 253-254

11. Birr, C. and Stollenwerk, V. (1979) Augewandte Chemie 18, 394-395

12. Itakura, K., Hirose, T., Crea, R., Riggs, A.D., Heyneker, H.L., Bolivar, F. and Boyer, H.W. (1977) Science 198 1056-1063

13. Goeddel, D.V., Kleid, D.G., Bolivar, F., Heyneker, H.L., Yansura, D.G., Crea, R., Hirose, T., Kraszewski, A., Itakura, K., Riggs, A.D. (1979) Proc. Natl. Aca. Sci. USA 76 106-110

14. Goeddel, D.V., Heyneker, H.L., Hozumi, T., Arentzen, R., Itakura, K. Yansura, D.G., Ross, M.J., Miozzari, G., Crea, R. and Seeburg, P. (1979) Nature 281, 544-548

15. Stawinski, Y., Hozumi, T., Narang, S.A., Bahl, C.P. and Wu, R. (1977) Nucleic Acids Res. 4, 353-372

16. Hirose, T., Crea, R. and Itakura, K. (1978) Tet. Letts., 2449-2452

17. Crea, R., Kraszewski, A., Hirose, R. and Itakura, K. (1978) Proc. Natl. Acad. Sci. USA 75, 5765-5769

18. Jay, E., Bembara, R., Padmanabhan, P. and Wu,R. (1974) Nucleic Acids Res. 1, 331-353

19. Maxam, A.M. and Gilbert, W. (1977) Proc.Natl. Acad. Sci USA 71 3455-3459

20. Bolivar, F.., Rodriguez, R.L., Greene, P.J., Betlach, M.C., Heyneker, H.L., Boyer, H.W., Crosa, J.H. and Falkow, S. (1977) Gene 2, 95-119

21. Backman, K., Ptashne, M. and Gilbert, W. (1976) Proc. Natl. Acad. Sci. USA 73 4174-4178

22. Gray, W.R. (1967) Methods Enzymol. 11, 469-475

23. Konigsberg, W. (1967). Methods Enzymol. 11, 461-469

24. Dwulet, F.E. and Gurd, F.R.N. (1976), Anal. Biochem. 76. 530-538

25. Smithies, O., Gibson, D., Fanning, E.M., Goodfliesh, R.M., Gilman, J.G. and D.L. Ballantyne. 1971, Biochemistry, 10, 4912-4921

26. Hu, S.-K., Low, T.L.K. and Goldstein, A.L. 1980, Fed. Proc. (In press).

27. Hu, S.-K., Low, T.L.K. and Goldstein, A.L. Submitted for publication.

28. Thurman, G.B., Rossio, J.L. and Goldstein, A.L. 1977. IN: Regulatory Mechanisms in Lymphocyte Activation, ed. D.O. Lucas, Academic Press, New York, p. 629

29. Arditti, R.R., Scaife, J. and Beckwith, J. (1968) J. Mol. Biol. 38, 421-426

30. Poliski, B., Bishop, R.J. and Gelfand, D.H. (1976) Proc. Natl. Acad. Sci. USA 73 3900-3904

31. Chang, L.M.S. (1971) Biochem. Biophys. Res. Commun.

44. 124-131.

32. "Interim Report of the Subcommittee on Biological Response Modifiers (BRM) of the DCT Board of Scientific Counsellors of the National Cancer Institute September 30, 1979, as reported in The Cancer Letter 5, 3-6 (1979).

Thymosin fraction 5 is a partially purified bovine thymic preparation containing over 30 peptides with molecular weights ranging from 1,000 to 15, 000 (1). Fraction 5 has been demonstrated to be an effective immuno-potentiating agent and can act in lieu of the thymus gland to reconstitute immune function in immunosuppressed animals and in humans with a number of primary and secondary immunodeficiency diseases (2). Thymosin alpha 1, a purified polypeptide component of this preparation, possesses a subset of fraction 5 activities associated with helper and amplifier functions and is a potent inducer of several of the later stages in T cell differentiation (3-5). These results suggest, and limited Phase I and Phase II clinical trials tend to confirm, that thymosin alpha 1 and fraction 5 may be useful restorative therapeutic agents in the treatment of immunodeficiency diseases (6,7) and immunosuppressed conditions (7,8) associated with thymic malfunction.

Thymosin alpha 1 is an acidic polypeptide (pl 4.2, MW 3, 108) of 28 amino acid residues blocked at the N-terminus by an acetyl group (9). The amino acid sequence of human thymosin alpha 1 (3) is probably identical to the bovine sequence (4,9) (Figure 1). With the elucidation of the sequence, it became possible to synthesize thymosin alpha 1 by chemical means (10,11).

US patent 4,079,127 to Goldstein et al describes and claims thymosin alpha 1, as purified from Fraction 5, as having the amino acid sequence:

(N-acetyl)-Ser-Asp-Ala-Ala-Val$^{5}$-Asp-Thr-Ser-Ser-Glu$^{10}$-Ile-Thr-Thr-Lys-Asp$^{15}$-Leu-Lys-Glu-Lys-Lys$^{20}$-Glu-Val-

Val-Glu-Glu$^{25}$-Ala-Glu-Asn-COOH

In the related synthetic work, US patent 4,148,788, the naturally sequence and the N-terminal acetyl group are duplicated in seeming recognition, proven wrong by the present invention, of the essentiality of that group to the bioactivity of the molecule.

According to the present invention a non-acetylated version of thymosin alpha 1 namely, desacetylthymosin alpha 1 is made available by expression from specially constructed strains of *E. coli* and shown to exhibit essentially the same bioactivity as naturally derived, N-acetylated thymosin alpha 1 in *in vitro* testing, and essentially identical RIA activity, such that the novel product of the invention is seen as a candidate for administration in the therapy of immuno-incompetent and immuno-suppressed patients.

The manner in which these and other objects and advantages of the invention are achieved will more fully appear from the detailed description which follows and from the accompanying drawings, in which:

Figure 1 depicts the design of a gene for desacetylthymosin alpha 1. The synthesis of the double stranded DNA involved the 16 oligonucleotides ($T_1$ through $T_{16}$) indicated by double headed arrows. The 5' ends have single-stranded cohesive termini to facilitate joining to plasmids cleaved with Eco R1 and Bam HI. A Bgl II site in the center of the gene assists in the analysis of recombinant plasmids. The Met codon ATG is inserted at the N-terminus to allow CNBr cleavage of desacetylthymosin alpha 1 from the expressed chimeric protein.

Figure 2 depicts the synthetic route to fragment $T_{15}$ starting from fully protected trideoxyribonucleotides.

Figure 3 summarizes litigation reactions for the gene and plasmid constructions described infra. Heavy dots indicate 5'-phosphate groups. Gene fragments are not drawn to scale.

Figure 4 depicts successive purifications of desacetylthymosin alpha 1 made in E. coli, thus: (a) DEAE elution; (b) gel filtration of the main DEAE peak; and (c) HPLC analysis and purification of the gel filtration pool.

Figure 5 is a thermolysin peptide map of desacetylthymosin alpha 1 from E. coli.

Figure 6 depicts dose/response curves in radioimmunoassay (Methods) for chemically synthesized N-acetyl thymosin alpha 1 (.--.--.), N-unblocked thymosin alpha 1 from E. coli extract (Δ--Δ--Δ), and N-acetyl thymosin alpha 1 isolated from bovine thymosin fraction 5 (o--o--o). Parallel responses indicate that complete immunochemical identity is shared by the three preparations. The lines represent graphs generated by a computer utilizing a program for a four-parameter logistics method.

## ABBREVIATIONS

In the specification and drawings certain abbreviations employed are as follows: T cell, thymus-dependent lymphocyte; TPSTe, 2, 4, 6-triisopropylbenzenesulfonyltetrazole; BSA, benzene sulfonic acid; tlc, thin layer chromatography; HPLC, high performance liquid chromatography; MIF, macrophage migration inhibitory factor; TdT, terminal deoxyribonucleotidyl transferase; RIA, radioimmunoassay; PBL, peripheral blood lymphocytes; PEC, peritoneal exudate cells; 3-SPITC, 3-sulfophenylisothiocyanate; DMSO, dimethyl sulfoxide; DMT, 4,4'-dimethoxytrityl; CE, 2-cyanoethyl; R, p-chlorophenyl; Bz, benzoyl; An, anisoyl; iBu, isobutryl; Py, pyridine; AcOH, acetic acid; $Et_3N$, triethylamine.

## DETAILED DESCRIPTION OF THE INVENTION

The synthesis in E. coli of human somatostatin and human insulin A and B chains in the form of CNBr-cleavable fusion proteins has previously been reported, as has the direct expression of human growth hormone (respectively, 12, 13, 14). As will appear from the detailed description which next follows, the former technique has been extended in the present work to the production of desacetylthymosin alpha 1.

All work involving recombinant DNA in E. coli was performed at Genentech in accordance with current NIH guidelines in certified host vector systems. Cloning and growth of cultures up to 10 liters was with derivatives of plasmid pBR322 in E. coli K12 (P3/HV1 containment).

## DETAILED DESCRIPTION

### EXPERIMENTAL PROCEDURES

#### Materials

Analytical reagents were used for the chemical synthesis of DNA. Pyridine was dried over KOH, distilled and stored over molecular sieves (Type 4A). TPSTe was synthesized as described in (15). Fully protected trideoxyribonucleotides were synthesized as described in (16). Sources of bacterial strains and enzymes were as described previously in (13). HPLC was performed on Spectra-Physics SP-8000 Liquid Chromatographs. Water for peptide HPLC was distilled from glass and passed through a column of Merck LiChrosorb RP-8 resin (25-40 μm). Solvents and reagents for buffers were HPLC grade.

### METHODS

#### Design of the Desacetylthymosin alpha 1 Gene

The synthetic gene for desacetylthymosin alpha 1 (Figure 1) was based on the known amino acid sequence (9). The primary nucleotide sequence was derived from the restrictive criteria applied previously (12) (ie., use of preferred prokaryotic codons, elimination of sequences with multiple complementarities, and minimization of AT or GC rich regions.) Additional features of the gene include: (a) the terminal restriction endonuclease site sticky ends, to facilitate incorporation into plasmids, (b) a methionine (ATG) codon placed just before the N-terminal codon to allow thymosin alpha 1 to be released from the chimeric protein by CNBr cleavage, (c) a central restriction site (Bgl II) for analysis of plasmids containing the gene, and (d) tandem stop codons.

Three considerations guided the division of each strand into the eight oligonucleotide fragments (10-15 bases) indicated in Figure 1: (a) Nucleotide overlaps of at least six base pairs with each of the opposing fragments of the complimentary strand were chosen for proper alignment during enzymatic ligation,(b) Each oligomer was synthesized using the minimum number of chemical condensations and (c) the overall synthesis required a minimum variety of trideoxyribonucleotide building blocks.

Synthesis of Fragment $T_{15}$,d(G-T-T-C-T-C-A-G-C-C-T-C)(12)

Oligodeoxyribonucleotides $T_1$ to $T_{16}$ were synthesized by a modified phosphotriester method using fully protected trideoxyribonucleotide building blocks,(12,17).

The synthesis is typified by the following procedure for fragment $T_{15}$ as summarized in Figure 2.

The fully protected trideoxyribonucleotides 4 (85 mg, 0.05 mmol) and 2(180 mg, 0.1 mmol) were deblocked at the 5' hydroxyls by treatment with 2% BSA in 7:3 (v/v) chloroform/methanol (10 and 20 ml, respectively) for 10 min at 0°C. Reactions were stopped by addition of saturated aqueous ammonium bicarbonate (2 ml), extracted with chloroform (25 ml) and washed with water (2 x 10 ml). The organic layers were dried (magnesium sulfate), concentrated to small volumes (about 5 ml) and precipitated by addition of petroleum ether (35°-60°C fraction). The colorless precipitates were collected by centrifugation and dried in a dessicator *in vacuo* to give 6 and 8, respectively,each homogeneous by silica gel tlc (Merck 60 F254, chloroform/methanol, 9:1).

Trimers 1 and 3 (270 mg, 0.15 mmol; 145 mg, 0.075 mmol) were converted into their phosphodiesters (5 and 7) by treatment with triethylamine/pyridine/water (1:3:1, v/v, 10 ml) for 25 min at R.T. Reagents were removed by rotary evaporation and the residues dried by repeated evaporations with anhydrous pyridine (3 x 10 ml). Trimer 8 (0.05 mmol) and trimer 7 were combined with TPSTe (50 mg, 0.15 mmol) in anhydrous pyridine (3 ml) and the reaction mixture left *in vacuo* at R.T. for two hours. Tlc analysis showed that 95 of the trimer 8 had been converted into hexamer product (visualized by detection of the DMT group by spraying with 10% aqueous sulfuric acid and heating at 60°C). The reaction was quenched by addition of water (1.0 ml) and the solvent evaporated under reduced pressure. After removal of pyridine by coevaporations with toluene, the hexamer was deblocked at the 5' position with 2%BSA (8 ml) as described above for trimers 4 and 2. The product (10) was purified on a silica gel column (Merck 60 H, 3.5 x 5 cm) by step gradient elution with chloroform/methanol (98:2 to 95:5, v/v). Fractions containing product 10 were evaporated to dryness.

Similarly, trimer 5 was coupled to 6 and the fully protected product directly purified on silica gel. This latter compound was deblocked at the 3' end by triethylamine/pyridine/water as described above to give fragment 9.

Finally, hexamers 9 and 10 were coupled in anhydrous pyridine (2 ml) with TPSTe (75 mg, 0.225 mmol) as the condensing agent. Upon completion (4 hr, R.T.) the mixture was rotary evaporated and the residue chromatographed on silica gel. Product 11 (160 mg) was obtained by precipitation with petroleum ether and appeared homogeneous on tlc. A portion of compound 11 (20 mg) in pyridine (0.5 ml) was completely deblocked by treatment with concentrated ammonium hydroxide (7 ml, 8 h, 60°C) and subsequent treatment in 80% acetic acid (15 min, R.T.). After evaporation of acetic acid, the solid residue was dissolved in 4% aqueous ammonium hydroxide (v/v, 4 ml) and extracted with ethyl ether (3 x 2 ml). The aqueous phase was concentrated to 1-2 ml and a portion applied to HPLC (Figure 2a) for purification of 12. The fractions corresponding to the major peak were pooled (ca. 2.0 $O.D._{254}$ units) and concentrated to about 5 ml. The final product 12 was desalted on Bio-gel P-2 (1.5 x 100 cm) by elution with 20% aqueous ethanol, reduced to dryness and resuspended in water (200 µl) to give a solution of $A_{254}$=10. The sequence of 12 was confirmed by two-dimensional sequence analysis.

Construction of the Desacetylthymosin alpha 1 gene and plasmids

The desacetylthymosin alpha 1 gene was assembled from the 16 synthetic oligo-nucleotides by methods previously described in detail for somatostatin (12), insulin (13) and growth hormone (14). Ten microgram quantities of oligonucleotides $T_2$ through $T_{15}$ were quantitatively phosphorylated with $[\gamma - ^{32}P]$ - ATP (New England Nuclear) in the presence of $T_4$ polynucleotide kinase (13) to give specific activities of approximately 1 Ci/mmol. Radiolabelled fragments were purified by 20% polyacrylamide/7 M urea gel electrophoresis and sequences of the eluted fragments were verified by two-dimensional electrophoresis/homochromatography (18) of partial snake venom digests. Fragments $T_1$ and $T_{16}$ were left unphosphorylated to minimize undesired polymerization during subsequent ligation reactions. These oligonucleotides (2 μg each) were assembled in four groups of four fragments by $T_4$ DNA ligase using published procedures (13) (see Figure 3). The reaction products were purified by gel electrophoresis on a 15% polyacrylamide gel containing 7 M urea (19). The four isolated products were ligated together and the reaction mixture resolved by 10% polyacrylamide gel electrophoresis. DNA in the size range for the thymosin alpha 1 gene (90-105 base pairs) was electroeluted.

Plasmid pBR322 (20)(0.5 μg) was treated with Bam HI and Eco RI restriction endonucleases and the fragments separated by 10 polyacrylamide gel electrophoresis. The large fragment was recovered from the gel by electroelution and subsequently ligated to the assembled synthetic DNA (14). This mixture was used to transform E. coli K12 strain 294 (21), ATCC No. 31446. Plasmid DNA was prepared from ampicillin-resistant, tetracycline-sensitive transformants (13). These recombinant plasmids were screened for the presence of a unique Bgl II restriction endonuclease site, the Eco RI/Bam HI inserts analysed for the expected size, and the inserted DNA sequenced by the Maxam-Gilbert technique (19). An Eco RI fragment promotor and β-galactosidase gene isolated from λ plac 5 L8UV5 DNA (29,30) containing the lac UV5 was ligated to the desacetylthymosin alpha 1 gene at the Eco RI site of plasmid pTh$\alpha_1$ in the correct orientation to give pTh$\alpha_1$β gal (13).

Growth of Culture

*E. coli* strain 294/pTh$\alpha_1$β gal was grown to late log phase ($A_{600}$ = 2.0) in ten liters of LB medium with 20 mg/l ampicillin in a ten liter fermentor (New Brunswick Scientific) at 37°C. Cells were killed by incubation in the fermentor with 0.25% each of toluene and phenol for 30 min, then collected by centrifugation and frozen. Yield = 30 g wet cells.

Purification of Desacetylthymosin alpha 1

Purification steps were monitored by radioimmunoassay (see below) and HPLC (Figure 4c).

Twenty four grams of thawed cells were lysed by sonication at 4°C in 135 ml lysis buffer (10% sucrose, 0.2 M sodium chloride, 50 mM EDTA, 0.1 M TRIS-HC1 pH 7.9, 0.1 mM phenylmethylsulfonylfluoride). The lysate was centrifuged (7000 rpm, 20 min) and the pellet suspended in 100 ml 7M guanidine hydrochloride by stirring overnight at 4°C. This slurry was centrifuged (7000 rpm, 30 min) and the supernatant added to ten volumes of cold water. The precipitate (1.8 g dry weight) was collected by centrifugation (4000 rpm, 30 min) and shown to contain the chimeric protein by SDS gel electrophoresis. This material was suspended in 200 ml 88% formic acid, 1.6 g CNBr (Sigma)(40-fold molar excess over total methionine) added, and the stoppered mixture was stirred overnight at R.T. in a high draft hood. The resulting solution was rotary evaporated (oil pump with dry ice and NaOH traps in a high draft hood) at 30°C, and the residue suspended in 7.5 M urea (10 ml), adjusted to pH 5 with ethanolamine and stirred two hours at R.T. This solution was diluted with cold water (200 ml) and stored at 4°C overnight, resulting in the precipitation of a large percentage of contaminating proteins. After centrifugation (9000 rpm, 35 min) the supernatant containing thymosin alpha 1 was loaded onto a DEAE cellulose column (Whatman DE-52 2.5 x 60 cm) equilibrated at R.T. with 20 mM ammonium bicarbonate, pH 7.9 and eluted with a linear gradient of 0.0 to 0.5 M sodium chloride in the same buffer to give several RIA positive peaks. In figure 4a the data is depicted as follows: DEAE-cellulose eluted with a linear gradient of NaCl (........) as described in Methods, showing desacetylthymosin alpha 1 estimated by RIA (--------) and $A_{215}$(————). The major peak fractions were pooled, lyophilized and size fractionated on an Ultrogel AcA 202 column (1 x 100 cm) by elution with 10 mM ammonium bicarbonate, pH 7.9 (Figure 4b, with HPLC -estimated desacetylthymosin alpha 1 (--------) and $A_{215}$(————). The desacetyl thymosin alpha 1-containing fractions were further purified by preparative HPLC (Figure 4c) on a 0.4 x 30 cm LiChrosorb C-18, 10 μm (Merck) column at 35° with a resolving gradient from 6 to 21% acetonitrile (Fisher HPLC grade) in 50 mM ammonium acetate, pH 7.1 at 0.6% per min and a flow rate of 1.5 ml/min. About 0.2 mg desacetylthymosin alpha 1 (elution time 18.3 min) was purified in this run. By comparison, synthetic N-acetyl thymosin alpha 1 elutes at 18.7 min.

<u>Chemical Characterization</u>

<u>Amino Acid Analysis</u> - Samples were hydrolyzed with twice-distilled 6N HCl at 110° for 24 hr. in sealed evacuated Pyrex glass tubes. Analyses were performed with a Beckman--Spinco amino acid analyzer model 119 CL employing single column methodology on Beckman W-3 resin.

<u>Enzymatic Digestion</u> - Thermolysin digestion was performed in IM ammonium bicarbonate at pH 8.3 at 37°C. Thermolysin was added to the protein solution to a final ratio of enzyme to substrate of 1:50 (W/W). The enzymatic digest was lyophilized immediately after 2.5 hr.

<u>Peptide Mapping</u> - Thermolysin digests were separated by paper chromatography and electrophoresis as described in (9). Peptides were detected by staining with cadmium--ninhydrin reagent. To analyze the amino acid composition of the peptides, the unstained chromatogram was stained with fluorescamine. The fluorescent spots were cut out, eluted with water, hydrolyzed and analyzed.

Isolation and Purification of Peptides - Peptides used for sequence studies were purified by paper chromatography and/or electrophoresis as described in (9).

Manual Sequence Determination - The sequences of small peptides were determined by manual Edman degradation using dansyl-monitored Edman (22) or subtractive Edman procedures (23).

Automated Sequence Analysis - Automated amino acid sequence analysis was performed with a Beckman Sequencer (model 890 C) using the DMAA program (Beckman peptide program 102974). *E. coli* desacetylthymosin alpha 1 was precoupled with 3-SPITC (24) before the sequencer program was initiated. The products were identified by HPLC and by amino acid analysis after back-hydrolysis with hydriodic acid (HI)(25).

TdT Assay

The *in vitro* TdT suppression assay was performed as described by Hu et al. (26, 27).

A. Cell Preparation; Six to eight-week old Balb/c mice were sacrificed by cervical dislocation and the thymus glands removed. Cells were dispersed over a fine metal screen, aspirated through a 25 guage needle and spun at 250 g for 10 min. Cells were then resuspended in HEPES buffered RPM1-1640 (Gibco, Grand Island, NY) at 5 x $10^6$ cells/ml.

B. Incubation with Thymosin. Approximately $10^8$ cells were cultured with thymosin fractions or saline for 20 hours at 37°C. The cells were prepared in a sterile petri dish containing 20 ml RPM1-1640 supplemented with penicillin, streptomysin, L-glutamine, 25 mM HEPES and 10% fetal calf serum.

C. Enzyme Extraction. The cells were harvested and ruptured by a sonicator (Heat System-Ultrasonics, Inc. model W-225R) in potassium cacodylate buffer (pH 7.5). The cell homogenate was then ultracentrifuged at 40,000 rpm for 1 hour.

D. Enzyme Assay. 25 µl of the supernatant was incubated with 1 ml of assay reagents (0.2 M potassium cacodylate, 8 mM $MgCl_2$, 1 mM 2-mercaptoethanol, 0.02 mM $p(dA)_{100}$(Bethesda Research Lab, MD), 1 mM [8-$^3$H]-dGTP for 30 min. at 37°. 100 µl of the incubation mixture was removed and sprayed on to a GF/c filter. The filters were washed successively in 5% trichloroacetic acid containing 1% sodium pyrophosphate, 1N HCl, and 95% ethanol, dried (110°C, 10 min) and counted in a scintillation counter.

One unit of enzyme activity is defined as the amount catalyzing the incorporation of 1 pmol of [8-$^3$H]-dGTP into acid insoluble material in one hour at 37°C. Specific activity was calculated from the enzyme activity per $10^8$ viable cells.

<u>MIF Assay</u>

A. <u>Preparation of Cells</u>. The MIF assay is a modification of a method reported previously by Thurman et al. (28). Hartley guinea pigs (400-600 gm) were injected intradermally with 0.1 ml of Freund's complete adjuvant (3113-60, Difco, Detroit, MI) containing 10 µg of preservative-free tuberculin purified protein derivative (PPD-CT68, Connaught Labs, LTD, Willowdale, Toronto, Canada). Ten days later, the animals were thymectomized. Animals were bled by cardiac puncture 48 or 72 hours postsurgery, and peripheral blood mononuclear cells (PBL) were obtained by centrifuging the heparinized blood through a Ficoll-metrizoate gradient (Lymphoprep, Nyegaard and Co.) at 400 x g for 40 minutes at 20°C. The cells recovered from the interface were washed twice in HRPMI medium (RPMI-1640 with 25 mM HEPES) containing 100 µg/ml streptomycin and 100 U/ml penicillin and adjusted to 5 x $10^6$ cells/ml. Rate-frozen PBL (-1°C/min, 9%DMSO), maintained for 4 weeks at -70°C, were thawed washed and utilized in a similar manner. Peritoneal exudate cells (PEC) were obtained from nonsensitized guinea pigs injected intraperitoneally 3 to 5 days previously with 20-30 ml of sterile light mineral oil using a 23 gauge needle. These cells were washed 3 times to remove residual oil and adjusted to 45 x $10^6$ cells/ml. The PBL were mixed with the PEC at a 1:9 ratio by adding one volume of PBL to one volume of PEC, and a migration inhibitory factor (MIF) assay performed using the microdroplet technique described below.

B. MIF Microdroplet Thymosin Assay. To a stock solution of 0.45% Sea Plaque Agarose (Microbiological Associates, Bethesda, MD), that had been melted in boiling water, an equal volume of 2X HRPMI was added, yielding a 0.225% agarose in HRPMI solution. This was maintained at 37°C in water bath. The PBL-PEC cell mixture was centrifuged at 200 x g for 10 minutes. All supernatant fluid was removed and the cell button adjusted to 37°C in a water bath. One microliter of the 0.225% agarose per $10^6$ cells (e.g. 100 µl per $10^8$ cells) was added to the cell button and cells were resuspended using a 50 µl Hamilton Syringe in a Hamilton Repeating Dispenser (Hamilton Co., Reno, Nev.) Cell agarose droplets, one µl in size were placed in the center of wells of an unprocessed Costar microtiter plate (Microbiological Associates, Bethesda, MD). The droplets were allowed to solidify at 20°C for 10-20 minutes in a humidified atmosphere. HRPMI was then added (containing either no additives, PPD, thymosin, or thymosin and PPD) to the microtiter plates to bring the total volume to 100 µl/well. PPD was added at 5 µg/ml, and thymosin preparations at various concentrations. Following a 24 hour incubation at 37°C in a human atmosphere (5Δ $CO_2$ in air), the areas of migration were measured using a B and L Tri-Simplix Microprojector. The areas of migration are considered to be ellipsoid in shape. The major and minor axes were measured, and the areas of each ellipsoid computed. The major and minor axes of the agarose droplet were subtracted from the total area to give the area of cellular migration. The means and standard errors of the areas of migration of replicate cultures were computed. The percent specific inhibition was calculated using the formula given below.

$$PSI = 100 - \left[ \frac{\text{Area (thymosin +PPD)}}{\text{Area (thymosin)}} \times \frac{\text{Area (HRPMI)}}{\text{Area (PPD)}} \times 100 \right]$$

Radioimmunoassay for Thymosin alpha 1 - A detailed description of the radioimmunoassay for thymosin alpha 1 is in preparation (McClure, Lameris, Wara, and Goldstein). Antiserum to synthetic thymosin alpha 1, produced in rabbits by immunization with the synthetic peptide coupled to hemocyanin by means of glutaraldehyde, was used in the RIA without purification and at final dilutions between 1:10,000 and 1:20,000. Labelled thymosin alpha 1 was prepared by radioiodination (immobilized lactoperoxidase, Enzymobead Radioiodination Kit, Bio-Rad, Richmond, CA) of a synthetic, tyrosine-containing analog, $(Tyr^1)\alpha_1$. Labelled thymosin alpha 1 of high specific activity (150-200 Ci/µg) was purified by gel filtrations on Sephadex G-10 and G-25 and found to retain immunoreactive integrity as assessed by incubation with excess antibody. The assay conditions included incubation of standard or unknown with antiserum in phosphate-buffered saline for 1 hour, addition of radioactive thymosin alpha 1, and successive incubations at 37°C (1 hour) and 4°C (48 hours). Second antibody (goat anti-rabbit gamma globulins) was used to precipitate the immune complexes formed during competitive binding. By using the highly purified synthetic thymosin alpha 1 standard, a calibration curve was generated which had a minimal detectable dose of 30-50 pg and an $E.D._{50}$ (50 response) of 150-400 pg. Measurements of the radioactivity bound in the immunoprecipitate were reduced by a four-parameter logistics program to a graph of counts bound at defined dose divided by counts bound at zero dose (Bi/Bo X 100) versus $\log_{10}$ dose of peptide.

RESULTS

Synthesis of Oligodeoxyribonucleotides

Oligomers $T_1$ to $T_{16}$ were synthesized as described

for fragment $T_{15}$ in Methods using the fully protected trimer building blocks listed in Table I.

The fully protected oligomeric products were deblocked and purified by HPLC (example, Fig. 2a). Each purified oligomer was 5'-phosphorylated and its size and sequence confirmed as described above for fragment $T_{15}$. Table II is a list of the oligomers synthesized in this way.

TABLE I. TRIMER LIBRARY FOR DESACETYLTHYMOSIN $\alpha_1$ SYNTHETIC GENE

| | | Rf a | Rf b | Present in | | | Rf a | Rf b | Present in |
|---|---|---|---|---|---|---|---|---|---|
| 1. | AAT | 0.24 | 0.52 | $T_1, T_7$ | 20. | GAA | 0.15 | 0.39 | $T_6, T_{10}, T_{11}$ |
| 2. | AAC | 0.28 | 0.55 | $T_{12}, T_{14}$ | 21. | GAT | 0.22 | 0.49 | $T_4, T_9, T_{16}$ |
| 3. | AAG | 0.15 | 0.40 | $T_8, T_9, 2xT_{10}, T_{11}$ | 22. | GAC | 0.23 | 0.52 | $T_5, T_{14}$ |
| 4. | ATT | 0.26 | 0.56 | $T_{16}$ | 23. | GAG | 0.09 | 0.36 | $T_{11}, T_{12}$ |
| 5. | ATC | 0.28 | 0.58 | $T_8$ | 24. | GTT | 0.18 | 0.45 | $T_{15}$ |
| 6. | AGA | 0.24 | 0.50 | $T_7$ | 25. | GTA | 0.25 | 0.42 | $T_6$ |
| 7. | AGT | 0.14 | 0.40 | $T_7$ | 26. | GTC | 0.22 | 0.51 | $T_{11}$ |
| 8. | AGC | 0.19 | 0.48 | $T_{15}$ | 27. | GCA | 0.14 | 0.39 | $2xT_5$ |
| 9. | TAA | 0.17 | 0.44 | $T_4T_{12}$ | 28. | ATG | 0.11 | 0.32 | $T_5$ |
| 10. | TAC | 0.22 | 0.55 | $T_3, 2xT_4$ | 29. | ACA | 0.12 | 0.40 | $T_6$ |
| 11. | TTT | 0.24 | 0.53 | $T_8$ | 30. | AGT | 0.14 | 0.42 | $T_8$ |
| 12. | TTC | 0.26 | 0.52 | $2xT_3, T_{14}$ | 31. | TAG | 0.10 | 0.32 | $T_{12}$ |
| 13. | TCA | 0.28 | 0.53 | $T_1, T_5, T_6$ | 32. | TTC | 0.19 | 0.50 | $T_{14}$ |
| 14. | TGA | 0.12 | 0.37 | $T_2$ | 33. | TGA | 0.13 | 0.37 | $T_2, T_3$ |
| 15. | TGT | 0.13 | 0.41 | $T_1, T_2$ | 34. | CTT | 0.19 | 0.49 | $T_{13}$ |
| 16. | TGC | 0.18 | 0.47 | $2xT_2$ | 35. | CTC | 0.20 | 0.56 | $T_{15}$ |
| 17. | CTT | 0.25 | 0.55 | $T_9, 2xT_{13}$ | 36. | GAG | 0.04 | 0.22 | $T_9$ |
| 18. | CTC | 0.26 | 0.59 | $T_7, T_{13}, T_{15}$ | 37. | GTT | 0.07 | 0.29 | $T_{10}$ |
| 19. | CCT | 0.30 | 0.55 | $T_{16}$ | 38. | GCT | 0.10 | 0.35 | $T_{11}$ |

Trimers 1 to 27: 5'-O-dimethoxytrityl-3'-O-2-cyanoethyl-p-chlorophenylphosphate derivatives.
Trimers 28 to 38: 5'-O-hydroxyl-3'-O-anisoyl derivatives.
Thin layer chromatography (silica gel) was in methanol/chloroform: (a) System A, 0.5/9.5 v/v, (b) System B, 1.0/9.0 V/V.

TABLE II

SYNTHETIC OLIGONUCLEOTIDES FOR DESACETYLTHYMOSIN $\alpha_1$ GENE

| COMPOUND | SEQUENCE | LENGTH | RT* |
|---|---|---|---|
| $T_1$ | A-A-T-T-C-A-T-G-T-C | 10 | 17.4 |
| $T_2$ | T-G-A-T-G-C-T-G-C-T-G-T-T-G-A | 15 | 24.3 |
| $T_3$ | T-A-C-T-T-C-T-T-C-T-G-A | 12 | 20.3 |
| $T_4$ | G-A-T-T-A-C-T-A-C-T-A-A-A | 13 | 22.0 |
| $T_5$ | G-C-A-G-C-A-T-C-A-G-A-C-A-T-G | 15 | 24.8 |
| $T_6$ | G-A-A-G-T-A-T-C-A-A-C-A | 12 | 20.1 |
| $T_7$ | A-G-T-A-A-T-C-T-C-A-G-A-A- | 13 | 22.6 |
| $T_8$ | A-A-G-A-T-C-T-T-T-A-G-T | 12 | 20.2 |
| $T_9$ | G-A-T-C-T-T-A-A-G-G-A-G | 12 | 20.4 |
| $T_{10}$ | A-A-G-A-A-G-G-A-A-G-T-T | 12 | 21.1 |
| $T_{11}$ | G-T-C-G-A-A-G-A-G-G-C-T | 12 | 20.5 |
| $T_{12}$ | G-A-G-A-A-C-T-A-A-T-A-G | 12 | 20.4 |
| $T_{13}$ | C-T-T-C-T-T-C-T-C-C-T-T | 12 | 19.9 |
| $T_{14}$ | T-T-C-G-A-C-A-A-C-T-T-C | 12 | 20.5 |
| $T_{15}$ | G-T-T-C-T-C-A-G-C-C-T-C | 12 | 20.2 |
| $T_{16}$ | G-A-T-C-C-T-A-T-T-A | 10 | 17.2 |

* retention time (min) in HPLC analysis

Gene and Plasmid Construction

As shown in Figure 3, [$^{32}$P]- labelled oligomers were built into the desacetylthymosin alpha 1 gene by a series of T4 DNA ligase catalyzed reactions using the complementarity of overlapping fragments to insure proper ordering. Since fragments $T_1$ and $T_{16}$ contain restriction site sequences, they are self-complementary and could polymerize during the ligation reaction; to avoid this they were used in the unphosphorylated forms. The final ligation product was partially purified by electrophoresis on 10% polyacrylamide slab gel and elution of the region between 90 and 105 base pairs. This fraction was ligated into the large Eco RI-Bam HI fragment of pBR322 and transformed into E. coli K12/294. Five per cent of the transformation mixture was plated on LB plates containing 20 µg/ml ampicillin. The four ampicillin resistant colonies obtained were sensitive to tetracycline, suggesting insertion into the tetracycline resistance gene. Analysis of the plasmids from these four colonies showed that in each case the plasmid contained (a) a Bgl II site not found in pBR322 itself, indicating the presence of the thymosin alpha 1 gene (Fig. 1) and (b) a fragment of approximately 105 base pairs generated by Bam HI/Eco RI cleavage. The desacetylthymosin alpha 1 DNA sequence of one of these plasmids (pTh$\alpha_1$) was confirmed by Maxam/Gilbert sequencing techniques (19). The Eco RI fragment of λ plac5 L8UV5 containing the UV 5 lac promotor mutation and most of the β-galactosidase gene (29,30) was inserted into the Eco RI site of pTh$_1$. Transformants of E. coli K12/294 were screened for blue colonies on X-gal indicator plates containing ampicillin (13). Restriction analysis confirmed the presence and proper orientation of the lac fragment in several of the selected colonies. Plasmid isolated from one of these colonies was designated pTh$\alpha_1$βgal.

Expression of Desacetylthymosin alpha 1

E. coli K12/294 containing pTh$\alpha_1$βgal was grown to an $OD_{600}$ of about 1.0 in Luria broth containing ampicillin and the pelleted cells were treated overnight with CNBr/formic acid. RIA analysis of the residues gave values in the vicinity of one μg desacetylthymosin alpha 1 per ml fermentation broth (about 100,000 molecules/cell). A control of E. coli K12/294 containing pBR322 gave RIA interference levels of about 0.01 μg/ml.

Purification of Desacetylthymosin alpha 1

Twenty-four grams (wet weight) of E. coli 294/-pTh$\alpha_1$ βgal cells were lysed and the chimeric protein partially purified as described above to give 1.8 g of dry weight protein. This preparation was cleaved with CNBr to give a mixture of fragments including thymosin alpha 1. Many of the contaminating fragments were eliminated by a precipitation step. The desacetylthymosin alpha 1-containing supernatant was further resolved by DEAE-cellulose chromatography (Figure 4a), Ultrogel AcA 202 chromatography (Figure 4b) and preparative HPLC (Figure 4c). The 4.5 mg desacetylthymosin alpha 1 obtained in this way was used for chemical and biological characterization.

The RIA profile of the DEAE column (Figure 4a) indicates the presence in small amounts of other RIA-positive peptides. Amino acid analysis of the largest of these minor peaks shows it to be a peptide containing a β-galactosidase fragment as well as thymosin alpha 1, consistent with incomplete CNBr cleavage.

Chemical Characterization

Desacetylthymosin alpha 1 synthesized in E. coli has an elution time (18.3 min) almost identical to that (18.7 min) of synthetic thymosin alpha 1 in the HPLC gradient described in Figure 4c, the difference due to the absence of the N-acetyl group in the former. The HPLC-purified material gave an amino acid analysis consistent with the proposed structure (Table III). Analysis of the intact molecule gave a sequence identical to natural thymosin alpha 1 at 26 identifiable positions (Table IV). The peptide map (Figure 5) of a thermolysin digest contained all the peptides found in authentic N-acetyl thymosin alpha 1, plus the N-terminal peptide which could not previously be visualized by amine-dependent stains. Amino acid analysis and N-terminal end group analysis on each of the eluted thermolysin peptides were consistent with the thymosin alpha 1 sequence.

TABLE III

AMINO ACID COMPOSITION* OF E. COLI DESACETYLTHYMOSIN $\alpha_1$

| | Determined | Expected |
|---|---|---|
| Lysine | 3.50 | 4 |
| Histidine | 0 | 0 |
| Arginine | 0 | 0 |
| Aspartic Acid | 4.10 | 4 |
| Threonine | 2.84 | 3 |
| Serine | 2.86 | 3 |
| Glutamic Acid | 6.24 | 6 |
| Proline | 0 | 0 |
| Glycine | 0 | 0 |
| Alanine | 3.13 | 3 |
| Valine | 2.37 | 3 |
| Methionine | 0 | 0 |
| Isoleucine | 0.89 | 1 |
| Leucine | 0.97 | 1 |
| Tyrosine | 0 | 0 |
| Phenylalanine | 0 | 0 |

* The data are presented as assumed number of residues per molecule. The molecular weight is assumed as 3,100. All values were from a 24-h hydrolysate in 6 N HCl at 110°C. No correction was made for threonine, serine, valine, isoleucine or leucine.

TABLE IV

AUTOMATED SEQUENCE ANALYSIS OF E.COLI DESACETYLTHYMOSIN $\alpha_1$

Partial structural analysis of E. coli desacetylthymosin $\alpha_1$ was obtained by automated sequence analysis using the DMAA program on a Beckman 890C sequencer. A total of 120 nmoles was applied.

| Cycle | Amino acid | Recoveries (nmoles) | | Cycle | Amino acid | Recoveries (nmoles) | |
|---|---|---|---|---|---|---|---|
| | | By HI hyd. | By HPLC | | | By HI hyd. | By HPLC |
| 1 | Ser | –[b] | – | 15 | Asp | 13.9 | ND |
| 2 | Asp | 32.8 | 29.0 | 16 | Leu | 11.7 | ND |
| 3 | Ala | 37.4 | 31.5 | 17 | Lys | 8.5 | ND |
| 4 | Ala | 32.4 | 32.2 | 18 | Glu | 11.0 | ND |
| 5 | Val | 25.1 | – | 19 | Lys | 9.2 | ND |
| 6 | Asp | 14.3 | 20.5 | 20 | Lys | 7.1 | ND |
| 7 | Thr | 15.7[c] | 2.5 | 21 | Glu | 8.8 | ND |
| 8 | Ser | 3.9[d] | – | 22 | Val | 5.1 | ND |
| 9 | Ser | 2.4 | – | 23 | Val | 6.0 | ND |
| 10 | Glu | 14.3 | 17.6 | 24 | Glu | 6.6 | ND |
| 11 | Ile | 17.8[e] | ND[f] | 25 | Glu | 6.9 | ND |
| 12 | Thr | 16.1 | ND | 26 | Ala | 4.3 | ND |
| 13 | Thr | 15.8 | ND | 27 | Glu | 1.9 | ND |
| 14 | Lys | 9.3 | ND | 28 | Asn | – | – |

a. Values for amino acids recovered after HI hydrolysis and analyzed on an amino acid analyzer. No correction was made for destruction of PTH derivatives.

b. Not identifiable.

c. Values for threonine by amino acid analysis were calculated as 2-aminobutyric acid.

d. Serine residues were calculated as alanine after HI hydrolysis.

e. Isoleucine values were calculated as the sum of isoleucine and alloisoleucine.

f. ND= Not determined.

TABLE V

AMINO ACID COMPOSITION* AND N-TERMINAL RESIDUE OF THERMOLYSIN PEPTIDES OF DESACETYLTHYMOSIN $\alpha_1$ FROM E. COLI

| | Th1 | Th2 | Th3 | Th4 | Th5 |
|---|---|---|---|---|---|
| Lysine | 2.40(3) | 1.02(1) | | | |
| Histidine | | | | | |
| Arginine | | | | | |
| Aspartic acid | | 0.91(1) | 0.98(1) | 0.91(1) | 0.98(1) |
| Threonine | | 1.82(2) | 0.85(1) | | |
| Serine | | | 1.46(2) | | 0.71(1) |
| Glutamic acid | 2.05(2) | | 1.12(1) | 2.93(3) | |
| Proline | | | | | |
| Glycine | | | | | |
| Alanine | | | | 1.63(1) | 2.00(2) |
| Valine | | | 1.21(1) | 1.54(2) | |
| Methionine | | | | | |
| Isoleucine | | 1.20(1) | | | |
| Leucine | 0.91(1) | | | | |
| Tyrosine | | | | | |
| Phenylalanine | | | | | |
| TOTAL | 6 | 5 | 6 | 7 | 4 |
| N-terminal** | Leu | Ile | Val | Val | Ser |

* Results from 6N HCl hydrolysates at 110° for 24 hr. Numbers in parentheses refer to the amino acid composition of expected thermolysin fragments.

** Determined by dansylation and/or subtractive-Edman procedure.

In radioimmunoassay, *E. coli* desacetylthymosin alpha 1 is indistinguishable from both bovine and synthetic N-acetyl thymosin alpha 1. The slopes of the curves for binding to rabbit anti-synthetic thymosin alpha 1 antibody (Figure 6) indicate that the three preparations share complete immunochemical identity.

Biological Activity

TdT Assay Although the biological function of TdT is not clear, its restricted localization in thymus and low levels in bone marrow cells but not in spleen or peripheral lymphocytes (31) makes it a specific marker for T cell differentiation and maturation. It has previously been shown (25) that thymosin fraction 5 and thymosin alpha 1 (both natural and chemically synthesized) are able to induce a marked decrease of TdT activity by various thymosin alpha 1 preparations. The results (Table VI) indicate that *E. coli* desacetylthymosin alpha 1 suppresses TdT activity in murine thymocytes in a similar manner to the chemically synthesized thymosin alpha 1 (10).

0035454

TABLE VI

SUPPRESSION OF TdT ACTIVITY BY CHEMICALLY SYNTHESIZED AND E.COLI DESACETYLTHYMOSIN $\alpha_1$ IN MURINE THYMOCYTES*

| Preparation | Concentration | % Decrease** (n g/ml) |
|---|---|---|
| Thymosin $\alpha_1$ | 0.02 | 20 |
| Chemically | 0.2 | 62 |
| Synthesized | 0.4 | 23 |
| Desacetylthymosin $\alpha_1$ | 0.02 | 8 |
| From Transformed | 0.2 | 55 |
| *E. coli* | 0.4 | 34 |

* Cells are incubated with thymosin fractions for 20 hours at 37° in HRPMI and 10% fetal calf serum. See Experimental Procedures for details.

** Decrease in TdT activity relative to cell culture without thymosin fraction.

<u>In vitro Induction of MIF by Desacetylthymosin alpha 1</u>. Table VII shows that desacetylthymosin alpha 1 synthesized in *E. coli* is also identical in activity to synthetic N-acetylated thymosin alpha 1 in the macrophage migration inhibition factor (MIF) assay. As can be seen in Table VII, the *E. coli* produced desacetylthymosin alpha 1 is about 10,000 times more active than thymosin fraction 5 and is active at concentrations as low as 10 ng/ml. This assay demonstrates the ability of an added factor, like thymosin alpha 1, to induce development of immature lymphocytes, as measured by their ability to respond to an antigenic challenge by releasing the lymphokine MIF. As expected, crude fraction 5 is much less active and antigen alone gave no MIF release.

TABLE VII

RECONSTITUTION OF MIF* PRODUCTION IN PERIPHERAL BLOOD LYMPHOCYTES (PBL) OF THYMECTOMIZED GUINEA PIGS BY THE ADDITION OF THYMOSIN $\alpha_1$

| Preparation | Concentration µg/ml | Inhibition (MIF Production) | | | |
|---|---|---|---|---|---|
| | | Exp 1 | Exp 2** | Exp 3 | Exp4** |
| Thymosin | 200 | 24.7 | 29.1 | 28.9 | 38.9 |
| Fraction 5 | 20 | 14.1 | 19.5 | 15.9 | 12.5 |
| (Lot C100496) | 2 | 9.5 | 12.1 | 15.5 | 1.7 |
| Thymosin $\alpha_1$ | 1 | 52.3 | 46.8 | 48.4 | 59.1 |
| Chemically | 0.1 | 34.6 | 37.3 | 39.7 | 51.4 |
| Synthesized | 0.01 | 11.0 | 27.2 | 25.5 | 33.4 |
| | 0.001 | N.T. | N.T. | 10.6 | 7.8 |
| Desacetyl-thymosin $\alpha_1$ | 1 | 50.8 | 48.2 | 53.2 | 39.7 |
| From transformed | 0.1 | 29.4 | 33.3 | 34.4 | 32.2 |
| *E. coli* | 0.01 | 27.5 | 27.6 | 16.0 | 23.5 |
| | 0.001 | N.T. | N.T. | 8.6 | 14.4 |
| No Thymosin, PPD only | | 4.7 | -3.4 | 5.0 | 5.0 |

* MIF Assay Microdroplet Technique

**Rate-frozen PBL (-1°C/min, 9% DMSO), maintained for 6 weeks at -70°C Viability = 75%

CONVERSION TO THYMOSIN ALPHA 1 (ACETYLATED)

The product of the invention, though bioactive in its own right, may be selectively acetylated to form thymosin alpha 1, by reaction with acetic anhydride in buffered aqueous media under conditions favoring acetylation of the alpha amino group and disfavoring acetylation of the epsilon amino groups of the polypeptide's lysine residues. Thus, approximately 70 yields of the N-terminal acetylated product were obtained by adjusting pH of a sodium acetate (ca. 2M)-buffered aqueous solution on non-acetylated product (ca. 0.4 millimolar) to pH 5.5, cooling to 0°C., and adding 4 successive increments of acetic anhydride (5 micrograms each) over the course of one hour. Following each addition, the reaction mixture is approximately one millimolar in anhydride concentration. Unreacted anhydride is slowly hydrolyzed to acetic acid, which is taken up by buffer. Purification is by preparative HPLC or, in the case of large scale production, preferably by ion exchange chromatography.

The compound of the invention, because capable of enhancing the immune response, will find wide application as an immunopotentiating agent in the treatment of immunoincompetent and immunosuppressed conditions in human and other subjects, ie, the same conditions which appear amenable to treatment with N-terminally acetylated thymosin alpha 1. The compound should prove particularly useful as an adjunct to treatment of the neoplastic diseases with cancer chemotherapeutic agents (ie, agents which are cytostatic or cytocidal for dividing cells), as a means of off-setting the immunosuppressive side-effects of such agents on the normal bodily defensive mechanisms.

Dosage will vary according to the effect desired and in general be identical to that in which naturally derived thymosin alpha 1 is administered, eg, effective immunopotentiating dosage, preferably from about 0.1 to about 1 mg per square meter of body surface area, most preferably from about 0.3 to about 0.9 mg/$M^2$. When used as an adjunct to other chemotherapy, as in the case of cancer chemotherapy agents, effective immunopotentiating dosage will, of course, depend upon the dosage of the latter agent(s) and the magnitude of the concomitant immunosuppression effect.

The compound of the invention will be administered in combination with a pharmaceutically acceptable excipient, eg, pyrogen-free distilled water, suitably buffered with, eg, sodium bicarbonate and containing other addends appropriate to the delivery systems employed. Thus, for example, the polypeptide may be combined with antioxidants, targeting agents, slow release adjuvants, carbohydrate and other stabilizing agents and so on. In the case of parenteral administration (ie, by injection), the compound may be administered in combination with serum albumin or other blood carrier proteins. Alternatively, the compound might be provided in combination with aerosol delivery means for, eg, nasal administration. Thus, an aerosol pump or propellant gas-containing vehicle could be employed, possibly in combination with effective amounts of vasodilation agents.

Most commonly, the polypeptide of the invention will be administered intravenously, intraperitoneally or, in the case of tumor treatment, intralesionally. In such case the product may be packaged in sterile ampoules, vials and multiple dose vials. The neoplastic diseases with cancer chemotherapeutic agents (ie, agents which are cytostatic or cytocidal for dividing cells), as a means of off-setting the immunosuppressive side-effects of such agents on the normal bodily defensive mechanisms.

CLAIMS

1. A polypeptide having the formula, proceeding from the N-terminal to carboxy-terminal end, of:

$NH_2$-Ser-Asp-Ala-Ala-Val-Asp-Thr-Ser-Ser-
Glu-Ile-Thr-Thr-Lys-Asp-Leu-Lys-Glu-
Lys-Lys-Glu-Val-Val-Glu-Glu-Ala-Glu-Asn-COOH

2. The polypeptide of claim 1 in solution in an injectable, pharmaceutically acceptable excipient.

3. The polypeptide of claim 1 in combination with a delivery device for administration in aerosol form.

4. Use of the compound according to claim 1 for the preparation of a medicament for treating immunoincompetent and immunosuppressed conditions.

5. Use according to claim 4, characterized in that the medicament is in the form of an aerosol or a composition for parenteral intralesional or intravenous administration.

6. A method of producing the polypeptide according to claim 1 which comprises preparing a microbial cloning vehicle comprising a gene coding for said polypeptide, transforming a microbial host with said cloning vehicle, expressing said polypeptide, and isolating the expressed polypeptide.

7. The polypeptide according to claim 1 when prepared by the process of claim 6.

8. A cloning vehicle comprising a gene coding for the polypeptide according to claim 1 suited for transformation of a microbial host and use therein for expressing said polypeptide.

9. A cloning vehicle according to claim 8 which is the plasmid pTh$\alpha_1$$\beta$gal.

10. A microorganism transformed with the cloning vehicle according to claim 8.

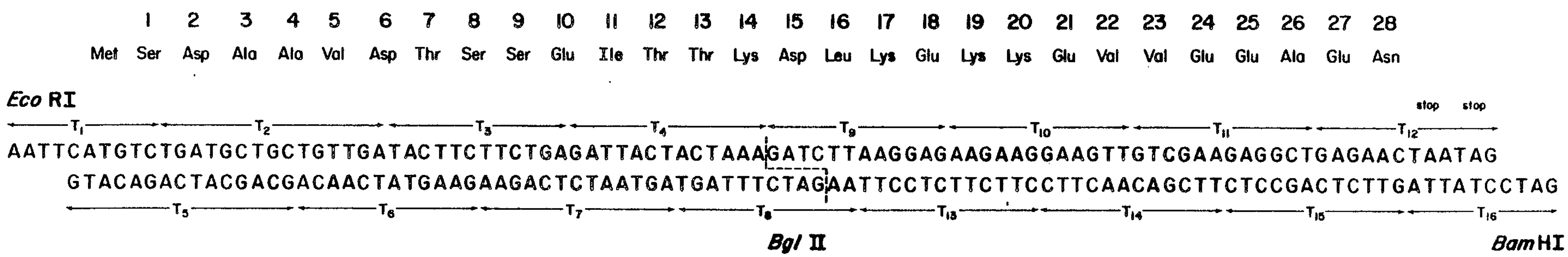

1 2 3 4 5 6 7 8 9 10 11 12 13 14 15 16 17 18 19 20 21 22 23 24 25 26 27 28
Met Ser Asp Ala Ala Val Asp Thr Ser Ser Glu Ile Thr Thr Lys Asp Leu Lys Glu Lys Lys Glu Val Val Glu Glu Ala Glu Asn
Eco RI
stop stop
T1 T2 T3 T4 T9 T10 T11 T12
AATTCATGTCTGATGCTGCTGTTGATACTTCTTCTGAGATTACTACTAAAGATCTTAAGGAGAAGAAGGAAGTTGTCGAAGAGGCTGAGAACTAATAG
GTACAGACTACGACGACAACTATGAAGAAGACTCTAATGATGATTTCTAGAATTCCTCTTCTTCCTTCAACAGCTTCTCCGACTCTTGATTATCCTAG
T5 T6 T7 T8 T13 T14 T15 T16
Bgl II
Bam HI


FIG. I.

0035454

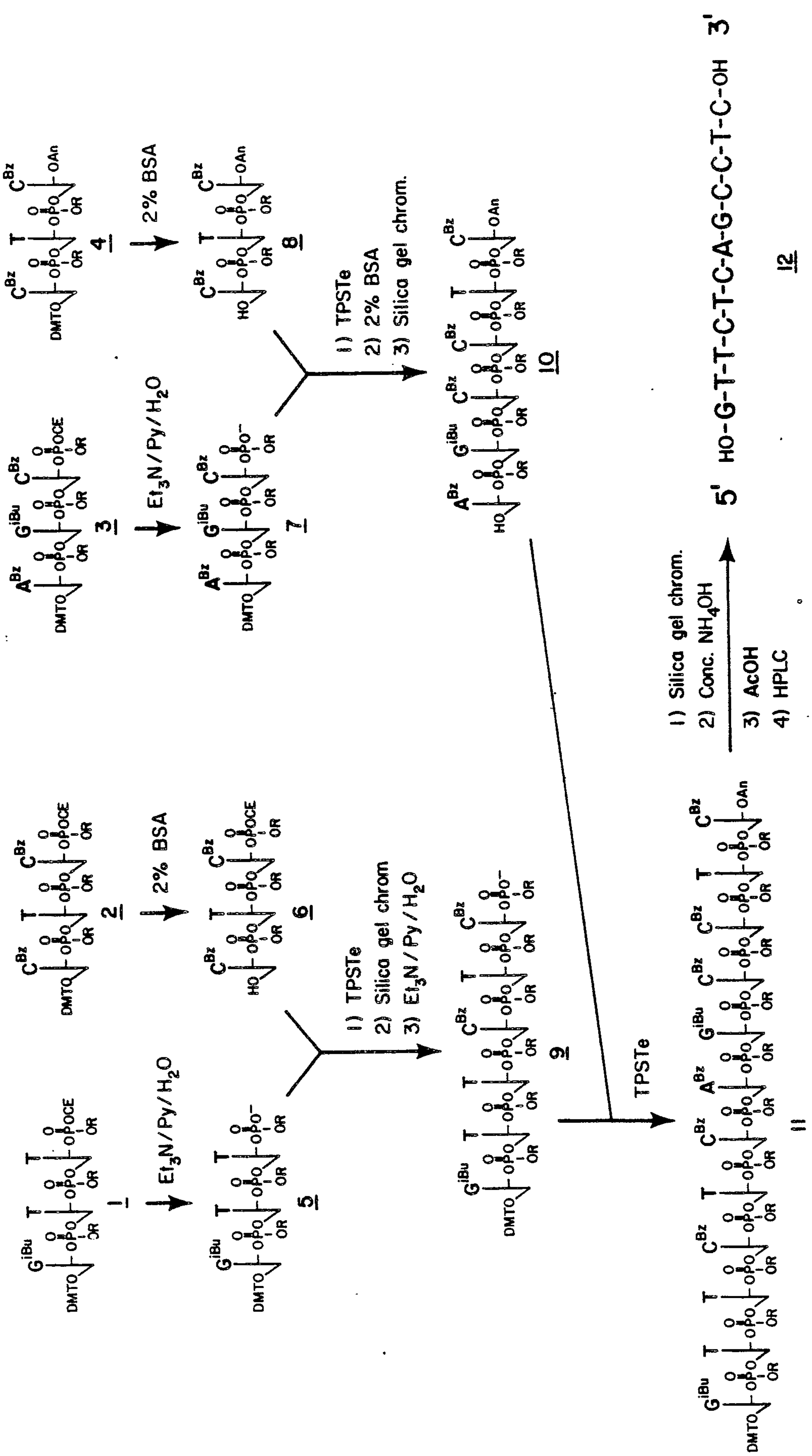


FIG. 2.

FIG. 3.

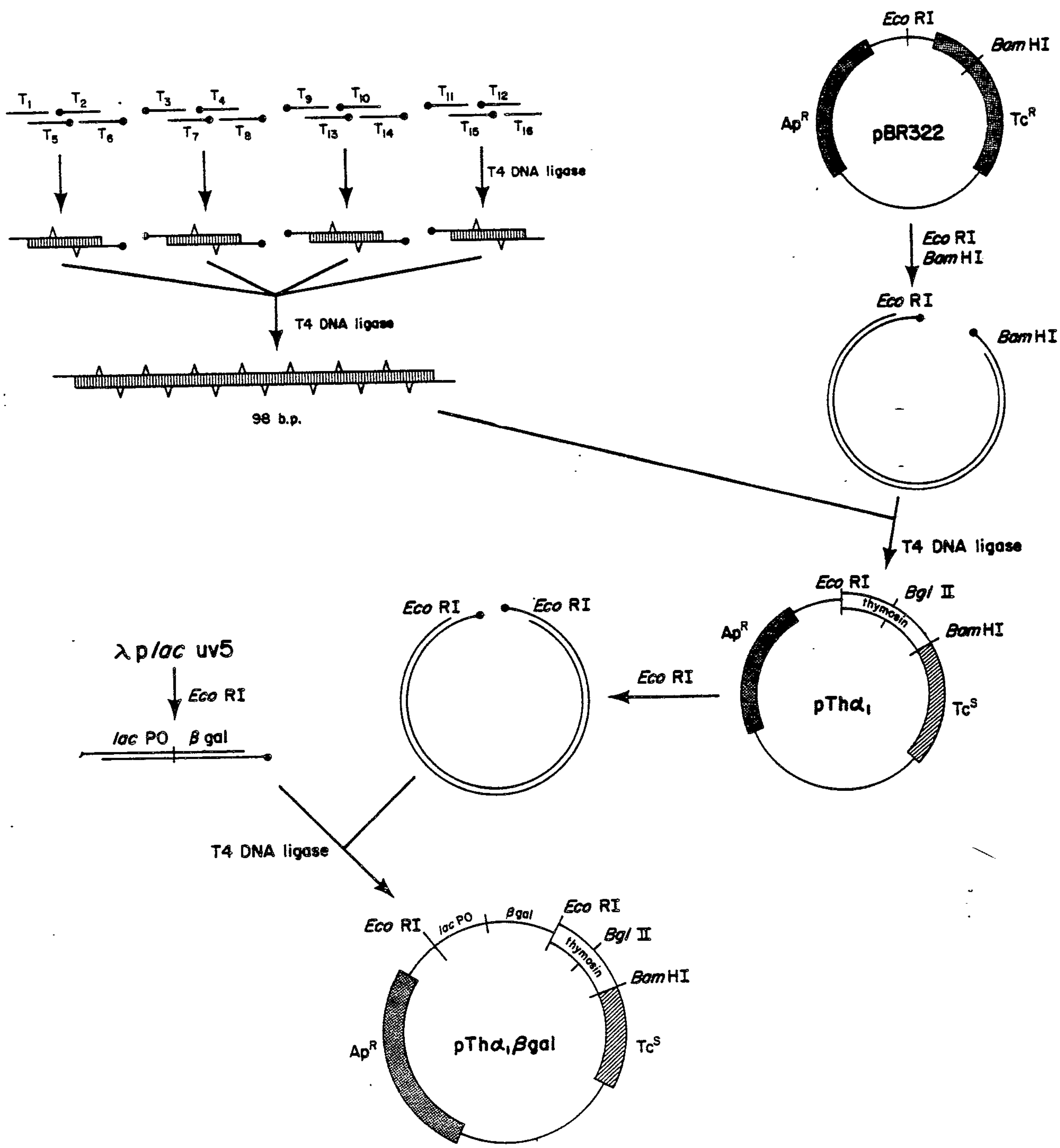

T1 T2 T3 T4 T9 T10 T11 T12
T5 T6 T7 T8 T13 T14 T15 T16
T4 DNA ligase
T4 DNA ligase
98 b.p.
Eco RI
Bam HI
ApR
pBR322
TcR
Eco RI
Bam HI
Eco RI
Bam HI
T4 DNA ligase
Eco RI
Bgl II
thymosin
Bam HI
ApR
pThα1
TcS
Eco RI
Eco RI
Eco RI
λ p lac uv5
Eco RI
lac PO
β gal
T4 DNA ligase
Eco RI
lac PO
βgal
Eco RI
Bgl II
thymosin
Bam HI
ApR
pThα1βgal
TcS

FIG. 4a.

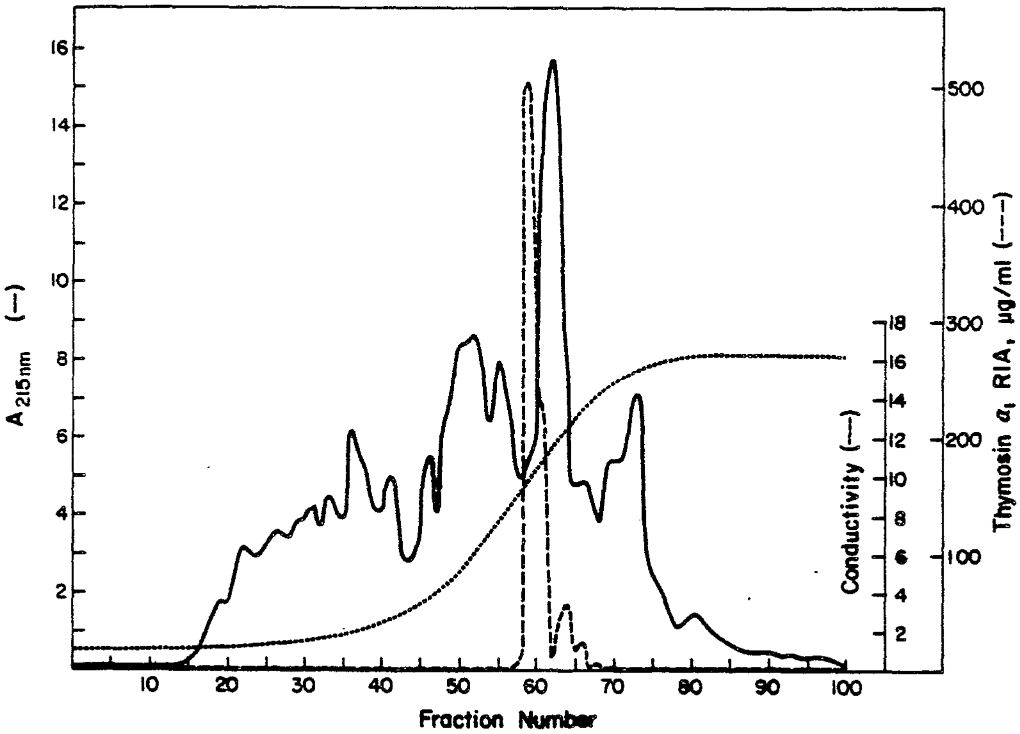


FIG. 4b.

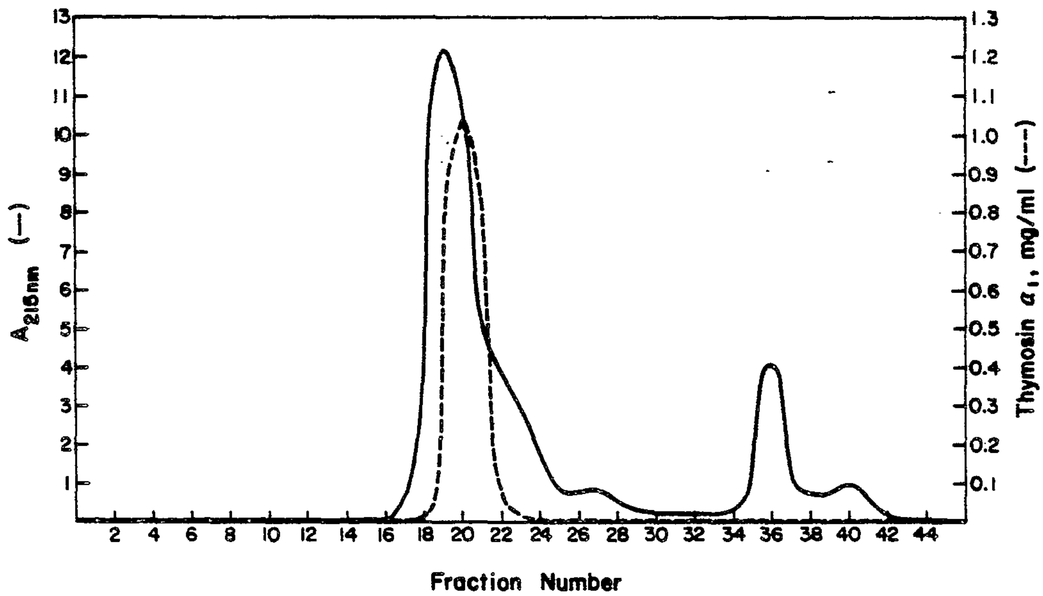


FIG. 4c.

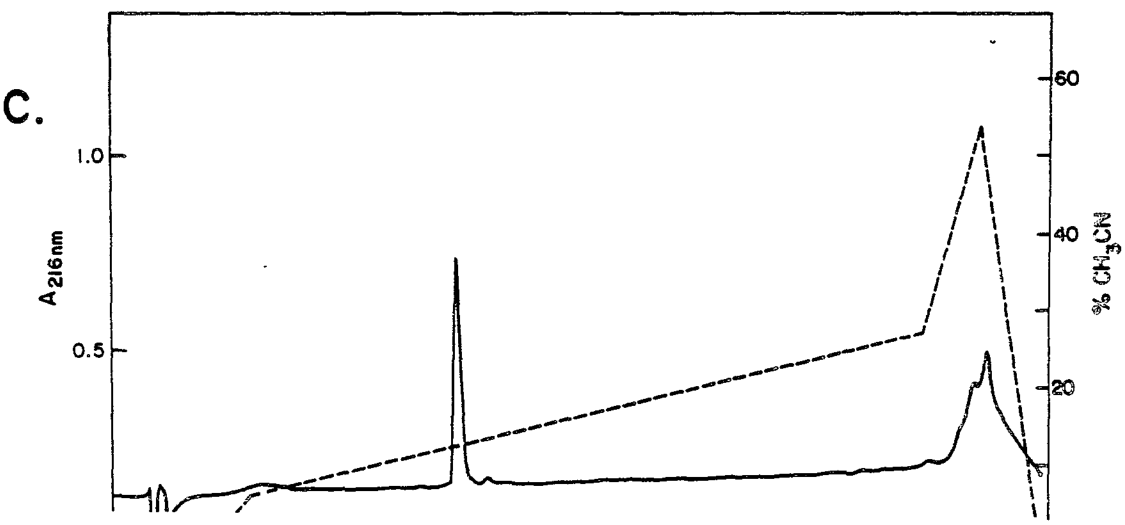

FIG. 5.

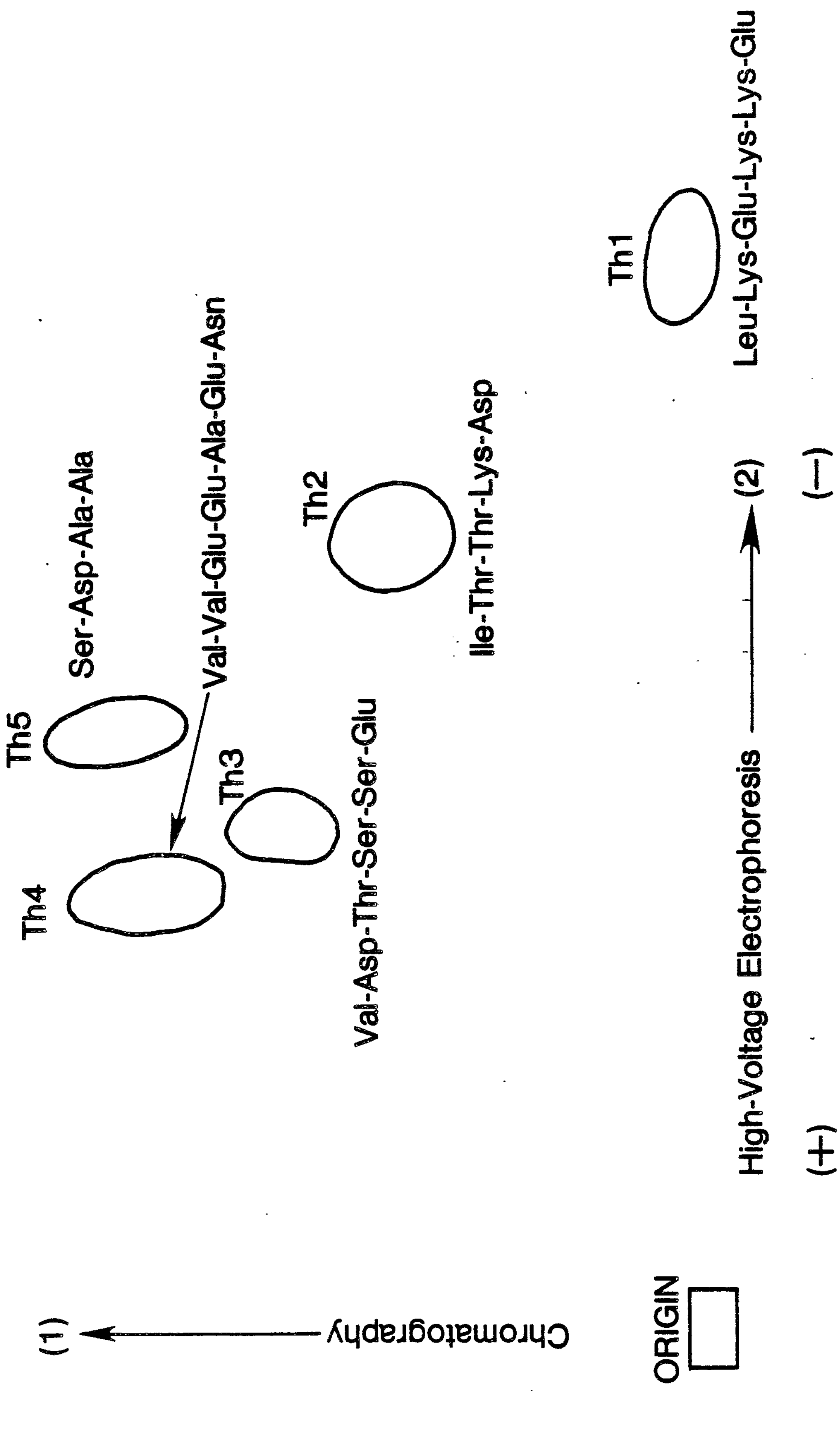

(1)
Chromatography
ORIGIN
Th4
Th5
Th3
Th2
Th1
Ser-Asp-Ala-Ala
Val-Val-Glu-Glu-Ala-Glu-Asn
Val-Asp-Thr-Ser-Ser-Glu
Ile-Thr-Thr-Lys-Asp
Leu-Lys-Glu-Lys-Lys-Glu
High-Voltage Electrophoresis
(2)
(+)
(−)